# EUROPEAN PATENT APPLICATION

(11) **EP 4 162 921 A1**
(43) Date of publication of application: **12.04.2023**
(21) Application number: 20939934.4
(22) Date of filing: 09.11.2020
(51) Int. Cl.: A61K 8/19, A61K 8/25, A61K 8/81, A61K 8/73, A61K 8/46, A61K 8/35, A61K 8/04, A61Q 17/04

(54) **WATER-DISPERSIBLE COSMETIC COMPOSITION FOR ULTRAVIOLET BLOCKING CONTAINING INORGANIC THICKENER AND ORGANIC THICKENER**

(30) Priority: 08.06.2020 KR 20200069288
(71) Applicant: Kolmar Korea Co., Ltd., Sejong 30004 (KR)
(72) Inventor: KIM, Jin Young, Seoul 07255 (KR); KIM, Jin Mo, Seoul 04188 (KR); HONG, In Ki, Seoul 06261 (KR)
(74) Representative: Gulde & Partner
(86) International application number: PCT/KR2020/015598
(87) International publication number: WO 2021/251566

(57) **Abstract**

The present invention relates to a water-dispersible cosmetic composition for ultraviolet blocking containing an inorganic thickener and an organic thickener, wherein the water-dispersible cosmetic composition for ultraviolet blocking can be frequently reapplied while having excellent adhesive strength to the skin and giving a cooling feeling and a refreshing feeling of use.

## Description

### Technical Field

The present invention relates to a sunscreen cosmetic composition in aqueous dispersion form containing an inorganic thickener and an organic thickener, and more particularly, to a sunscreen cosmetic composition in aqueous dispersion form, which has excellent skin adhesion and gives a cooling feeling and a refreshing feeling of use while being able to be reapplied frequently.

### Background Art

Recently, consumers' awareness of the need for sunscreen in daily life rather than sunscreen during summer or sports and during leisure activities has expanded. Sunscreen products used for this purpose are effective only for a limited period, and thus are recommended to be applied repeatedly every 2 to 3 hours. Therefore, the demand for UV protection products with a refreshing and light feeling of use has increased.

In general, sunscreen products may be manufactured in various forms such as cream, lotion, stick and powder types. Among them, the creams type that is sold a lot in the market contains a large amount of oil components, and thus has disadvantages in that it leaves a residual oily feeling and feels sticky, which limits frequent reapplication of the cream type. In addition, the cream type contains a large amount of an emulsifying agent, and thus when it comes into contact with water, a white cast phenomenon occurs in which it becomes white and milky while being reemulsified. In addition, the stick type contains no or tract amount of water, and thus when it is applied to the skin in summer, it is difficult to give a cooling feeling. Additionally, conventional sunscreen products have a problem of causing clumping or slipping when used together with makeup products.

Among the types of cosmetics, a gel or skin spray type may be a light and refreshing formulation, but it has a disadvantage in that it is difficult to incorporate a sufficient amount of a sunscreen agent while maintaining the formulation, because the formulation is light in weight. In addition, when a thickener is added to a water-soluble sunscreen agent, dehydration occurs, lowering the viscosity of the formulation. For these reasons, it is very difficult to realize a gel-type formulation which is easy to reapply while being an aqueous dispersion formulation containing a large amount of water.

Therefore, there is a need for the development of a sunscreen cosmetic composition in aqueous dispersion form, which may be continuously re-applied while having a fresh and light feeling of use, and may have improved skin adhesion and color durability without slipping even when used with makeup products.

### DISCLOSURE

### Technical Problem

The present invention has been made in order to solve the above-described problems, and an object of the present invention is to provide a sunscreen cosmetic composition in aqueous dispersion form, which has excellent skin adhesion and may be reapplied frequently while giving a cooling feeling and a refreshing feeling of use due to a large amount of water contained therein.

Objects of the present invention are not limited to the above-mentioned objects, and other objects not mentioned herein will be clearly understood by those skilled in the art from the following description.

### Technical Solution

According to one embodiment of the present invention, there may be provided a sunscreen cosmetic composition comprising a water-soluble sunscreen agent, and different kinds of thickeners which consist of a first thickener and a second thickener, wherein the first thickener is an inorganic thickener, the second thickener is an organic thickener, and the cosmetic composition is in aqueous dispersion form.

Preferably, the first thickener may be a silicate mineral.

Preferably, the second thickener may be at least one selected from among acrylic thickeners, polysaccharides, and derivatives thereof.

Preferably, the first thickener may include at least one selected from the group consisting of hectorite, bentonite, magnesium aluminum silicate, saponite, montmorillonite, smectite, kaolin, illite, saponite, and mixtures thereof.

Preferably, the second thickener may include at least one selected from the group consisting of carbomer, an acrylate/C10-30 alkyl acrylate crosspolymer, an acrylic acid/Vp crosspolymer, hydroxyethyl cellulose, hydroxypropyl cellulose, hydroxypropyl methyl cellulose, hydroxypropyl methyl cellulose stearoxy ether, cellulose gum, xanthan gum, tara gum, sclerotium gum, Acacia Senegal Gum, gellan gum, guar gum, ethyl cellulose, cellulose, carboxymethyl hydroxyethyl cellulose, carboxymethyl cellulose, methyl hydroxyethyl cellulose, hydrolysates thereof, and mixtures thereof.

Preferably, the water-soluble sunscreen agent may include at least one selected from the group consisting of terephthalylidene dicamphor sulfonic acid, phenylbenzimidazole sulfonic acid, disodium phenyl dibenzimidazole tetrasulfonate, benzophenone-4, benzophenone-5, and mixtures thereof.

Preferably, the content ratio of the water-soluble sunscreen agent to the different kinds of thickeners may be 1: 0.02 to 0.36.

Preferably, the content ratio of the first thickener to the second thickener may be 1: 0.1 to 30.0.

Preferably, the first thickener may be contained in an amount of 0.005 to 2.5 wt% based on the total weight of the composition.

Preferably, the second thickener may be contained in an amount of 0.3 to 3.0 wt% based on the total weight of the composition.

Preferably, the water-soluble sunscreen agent may be contained in an amount of 0.5 to 28.0 wt% based on the total weight of the composition.

Preferably, the composition may be in a gel form.

Preferably, the composition may have a viscosity of 35,000 to 150,000 cP.

### Advantageous Effects

The sunscreen cosmetic composition of the present invention is in aqueous dispersion form, is not sticky or greasy due to a large amount of water contained therein, and may give provide a cooling feeling and a refreshing feeling of use.

In addition, the sunscreen cosmetic composition of the present invention may be stably maintained at a viscosity equal to or higher than a certain level while the viscosity is not lowered by the water-soluble sunscreen agent, indicating that the composition may exhibit gel-type properties. Therefore, the composition has advantages in that it has good spreadability and is easy to reapply.

In addition, the sunscreen cosmetic composition of the present invention may not only give a soft feeling of use, but also have improved skin adhesion and color durability without clumping or slipping even when used with makeup products. In addition, the composition has an excellent sunscreen effect, and may exhibit a reduced degree of a residual oily feeling or a white cast.

### Brief Description of Drawings

In order to more fully understand the drawings referred to in the detailed description of the invention, a brief description of each drawing is presented, in which:

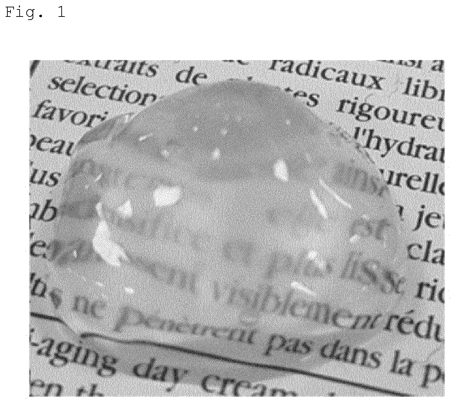

FIG. 1 is a view showing the appearance of a formulation of a composition of Example 1.

### Mode for Invention

Unless otherwise defined, all technical and scientific terms used in the present specification have the same meanings as commonly understood by those skilled in the art to which the present disclosure pertains. In general, the nomenclature used in the present specification is well known and commonly used in the art. In the following description of embodiments of the present invention, the detailed description of related publicly-known configurations or functions will be omitted when it may obscure the understanding of embodiments of the present invention. In addition, embodiments of the present invention will be described below, but the technical spirit of the present invention is not limited or limited thereto, and these embodiments may be modified and variously implemented by those skilled in the art.

The present invention relates to a sunscreen cosmetic composition in aqueous dispersion form containing an inorganic thickener and an organic thickener, which improve skin adhesion of the composition and prevent the viscosity of the composition from being reduced by a water-soluble sunscreen agent. The sunscreen cosmetic composition in aqueous dispersion form according to the present invention may contain a water-soluble sunscreen agent, an inorganic thickener, and an organic thickener.

Hereinafter, the components of the sunscreen cosmetic composition in aqueous dispersion form according to the present invention will be described in more detail.

In the composition of the present invention, the sunscreen agent is a component having a sunscreen effect, is water-soluble, and may be an organic sunscreen agent. The sunscreen agent may be mixed with and dispersed in a large amount of water due to its water-soluble properties, and when it is an organic sunscreen agent, it has excellent spreadability and causes no white cast.

The water-soluble sunscreen agent of the present invention may include at least one selected from the group consisting of terephthalylidene dicamphor sulfonic acid, disodium phenyl dibenzimidazole tetrasulfonate, phenylbenzimidazole sulfonic acid, benzophenone-4, benzophenone-5, and mixtures thereof.

In the present invention, the water-soluble sunscreen agent may be contained in an amount of 0.5 to 28 wt%, preferably 5 to 15 wt%, based on the total weight of the composition. In one embodiment, the water-soluble sunscreen agent may include 0.5 to 10.0 wt%, preferably 3 to 7 wt%, of terephthalylidene dicamphor sulfonic acid, 0.5 to 10 wt%, preferably 2 to 5 wt%, of disodium phenyl dibenzimidazole tetrasulfonate, and 0.5 to 8 wt%, preferably 2 to 5 wt%, of phenylbenzimidazole sulfonic acid, without being limited thereto.

If the composition of the present invention contains less than 0.5 wt% of the water-soluble sunscreen agent, the sunscreen effect will be insignificant, and if the composition contains more than 28 wt% of the water-soluble sunscreen agent, the formulation stability and feeling of use of the composition may be reduced.

The thickeners of the present invention serve to control the viscosity of the composition, and may include different kinds of thickeners which consist of a first thickener and a second thickener. Preferably, the first thickener may be an inorganic thickener, and the second thickener may be an organic thickener. The inorganic thickener and the organic thickener, which are contained in the composition of the present invention, have the effects of improving skin adhesion and preventing the viscosity of the composition from being reduced by the water-soluble sunscreen agent.

Specifically, in the present invention, the first thickener may be a silicate mineral. For example, the first thickener of the present invention may include at least one selected from the group consisting of hectorite, bentonite, magnesium aluminum silicate, saponite, montmorillonite, smectite, kaolin, illite, saponite, and mixtures thereof.

In the present invention, the first thickener may be contained in an amount of 0.005 to 2.5 wt% based on the total weight of the composition. If the first thickener is contained in an amount of less than 0.005 wt%, a problem may arise in that the viscosity of the cosmetic composition is not maintained at a certain level or higher, making it difficult to maintain the composition in a gel form, and if the first thickener is contained in an amount of more than 2.5 wt%, a problem may arise in that the feeling of use is not good because the soft feeling after drying is poor.

It is preferable that the thickeners of the present invention further include different kinds of a second thickener in addition to the first thickener.

The second thickener that may be used together with the first thickener may be at least one selected from among acrylic thickeners, polysaccharides, and derivatives thereof. For example, the second thickener of the present invention may be at least one selected from the group consisting of carbomer, an acrylate/C10-30 alkyl acrylate crosspolymer, an acrylic acid/Vp crosspolymer, hydroxyethyl cellulose, hydroxypropyl cellulose, hydroxypropyl methyl cellulose, hydroxypropyl methyl cellulose stearoxy ether, cellulose gum, xanthan gum, tara gum, sclerotium gum, Acacia Senegal Gum, gellan gum, guar gum, ethyl cellulose, cellulose, carboxymethyl hydroxyethyl cellulose, carboxymethyl cellulose, methyl hydroxyethyl cellulose, hydrolysates thereof, and mixtures thereof. In addition, the second thickener of the present invention may be added in a form (e.g., hydrolyzed sclerotium gum) made by hydrolyzing the above components with an acid, an enzyme, or other method.

In the present invention, the second thickener may be contained in an amount of 0.3 to 3.0 wt% based on the total weight of the composition. If the second thickener is contained in an amount of less than 0.3 wt%, a problem may arise in that the viscosity of the composition is very low, and thus a stable formulation cannot be formed, and if the second thickener is contained in an amount of more than 3.0 wt%, a problem may arise in that the viscosity of the composition is excessively high, and thus dispersion of the composition is difficult and the feeling of use is not good.

In the composition of the present invention, the content ratio of the first thickener to the second thickener may be 1: 0.1 to 30.0, preferably 1: 2.0 to 20.0, most preferably 1: 3.0 to 15.0. When the content ratio of the first thickener to the second thickener is within the above range, the composition has a high long-term formulation stability and an excellent feeling of use. In addition, in this case, the composition may exhibit a soft feeling of use, have improved skin adhesion, and exhibit a high sunscreen effect.

If the content ratio of the first thickener to the second thickener is less than 1: 0.1, a problem may arise in that the long-term viscosity stability and feeling of use of the composition are degraded, and if the content ratio of the first thickener to the second thickener is more than 1: 30, a problem may arise in that the formulation stability of the composition is degraded.

In general, the water-soluble sunscreen agent is an ionic material in which an anion of an acid and a cation of a base are coupled by electrostatic attraction. If the water-soluble sunscreen agent is added in a thickened state formed by dispersing and swelling the thickener in water, a problem arises in that dehydration occurs while the electrostatic attraction is broken, thus reducing the viscosity of the formulation. According to the present invention, it is possible to provide a sunscreen cosmetic composition in stable water dispersion form, the viscosity of which is not reduced even when the water-soluble sunscreen agent is added, by using the above-described types of inorganic thickener and organic thickener are used in combination.

In the composition of the present invention, the content ratio of the water-soluble sunscreen agent to the different kinds of thickeners may be 1: 0.02 to 0.36, preferably 1: 0.08 to 0.30, most preferably 1: 0.1 to 0.25. When the content ratio between the water-soluble sunscreen agent and the thickeners is within the above range, an aqueous dispersion gel formulation may be stably obtained, which has a high sunscreen function and excellent moisturizing ability. As such, the composition of the present invention may contain a high amount of the sunscreen agent, and thus has the advantage of exhibiting an excellent Sunscreen effect.

If the content ratio of the water-soluble sunscreen agent to the different kinds of thickeners may be less than 1: 0.02, a problem arises in that the composition is in a fluid liquid state due to its low viscosity and the feeling of use is degraded, and if the content ratio of the water-soluble sunscreen agent to the different kinds of thickeners may be more than 1: 0.36, a problem arises in that the thickeners are not easily dispersed due to high viscosity, the feeling of use and the formulation stability are degraded.

The water-soluble sunscreen cosmetic composition of the present invention may contain other ingredients commonly used in the art, for example, at least one selected from among a neutralizing agent, a moisturizing agent, a preservative, a functional additive, a skin conditioning agent, a film-forming agent, a fragrance, a lubricant, an antioxidant, a discoloration inhibitor, a stabilizer, and other additives, within a range that does not impair the effects of the present invention. For example, the composition according to the present invention may contain tromethamine as a neutralizing agent, butylene glycol as a moisturizing agent, phenoxyethanol as a preservative, and PVP, PVA, etc. as a film-forming agent. However, the present invention is not limited thereto, and the composition of the present invention may further contain various skin functional additives which are commonly used in the art and components which are contained in general cosmetic compositions.

The sunscreen cosmetic composition according to the present invention is in aqueous dispersion form. The sunscreen cosmetic composition of the present invention is an aqueous dispersion formulation containing 70 wt% or more of purified water, and may give a cooling feeling and a refreshing feeling of use due to a large amount of water contained therein.

The sunscreen cosmetic composition according to the present invention may be in a gel form. According to the present invention, it is possible to obtain a gel form, the viscosity of which may be stably maintained at a certain level, without lowering the viscosity, by adding the inorganic thickener and the organic thickener to the water-soluble sunscreen agent.

The viscosity of the sunscreen cosmetic composition according to the present invention may be 35,000 cP or more, preferably 35,000 cP to 150,000 cP, more preferably 40,000 cP to 90,000 cP. When the viscosity is within the above range, a stable gel formulation may be obtained.

As such, the present invention has the effect of being able to provide a sunscreen cosmetic composition in water-soluble gel form by adding the inorganic and organic thickeners to the water-soluble sunscreen agent to prevent a decrease in viscosity. The sunscreen cosmetic composition in water dispersion form according to the present invention may be stably maintained in a gel form for a long period of time and does not cause a residual oily feeling or a white cast. In addition, the composition of the present invention is easy to reapply frequently, gives a cooling feeling and a refreshing feeling of use due to a large amount of water contained therein, and may have improved skin adhesion and color durability without clumping or slipping even when used with makeup products.

Hereinafter, the present invention will be described in more detail with reference to examples, but the present invention is not limited to these examples.

### Preparation Example: Preparation of Examples and Comparative Examples

Sunscreen cosmetic compositions were prepared using the components shown in Table 1 below. Examples 1 to 3 are sunscreen cosmetic compositions prepared by changing the type and content of the second thickener, Comparative Examples 1 and 2 are sunscreen cosmetic compositions prepared by changing the ratio of the thickeners to the sunscreen agent, Comparative Examples 3 and 4 are sunscreen cosmetic compositions prepared by changing the ratio of the second thickener to the first thickener, Comparative Example 5 is a sunscreen cosmetic composition prepared by changing the type of the second thickener, Comparative Example 6 is a sunscreen cosmetic composition to which the second thickener was not added, and Comparative Example 7 is a sunscreen cosmetic composition to which the first thickener was not added.

**[Table 1]**

| Role | | Component | Ex. 1 | Ex. 2 | Ex. 3 | Comp. Ex. 1 | Comp. Ex. 2 | Comp. Ex. 3 | Comp. Ex. | 4 Comp. Ex. 5 | Comp. Ex. 6 | Comp. Ex. 7 |
|---|---|---|---|---|---|---|---|---|---|---|---|---|
| Purified water | | Purified water | 72.5 | 73 | 76 | 80 | 71 | 78 | 78 | 75 | 78 | 78 |
| Thickener | First thickener | Hectorite | 0.4 | 0.4 | 0.4 | 0.1 | 2.0 | 1.9 | 0.06 | 1.0 | 1.0 | - |
| | | Magnesium aluminum silicate | - | - | - | - | - | - | - | - | 1.0 | - |
| | Second thickener | Carbomer | 1.6 | 1.0 | - | 0.1 | 2.0 | - | - | - | - | - |
| | | Hydroxypropyl cellulose | - | 0.6 | 0.8 | - | 0.9 | - | - | - | - | 1.0 |
| | | Tara gum | - | - | 0.8 | - | - | 0.10 | 1.94 | - | - | 1.0 |
| | | PG-150/decyl alcohol/SMDI copolymer | - | - | - | - | - | - | - | 1.0 | - | - |
| | Total weight of thickeners | | 2.0 | 2.0 | 2.0 | 0.2 | 4.9 | 2.0 | 2.0 | 2.0 | 2.0 | 2.0 |
| Sunscreen agent | Terephthalylidene dicamphor sulfonic acid | | 7.0 | | | | | | | | | |
| | Disodium phenyl dibenzimidazole tetrasulfonate | | 3.0 | | | | | | | | | |
| | Phenylbenzimidazole sulfonic acid | | 3.0 | | | | | | | | | |
| Neutralizing agent | Tromethamine | | q.s. | q.s. | q.s. | q.s. | q.s. | q.s. | q.s. | q.s. | q.s. | q.s. |
| Moisturizing agent | Butylene glycol | | 3.0 | | | | | | | | | |
| Preservative | Phenoxyethanol | | q.s. | q.s. | q. s. | q.s. | q. s. | q.s. | q.s. | q. s. | q.s. | q. s. |

A specific preparation method is as follows.

Phase A was prepared by dispersing the first thickener and other additives such as a moisturizing agent in purified water using an AGI mixer at about 300 to 600 rpm. Phase B was prepared by dispersing the second thickener in purified water at 300 to 600 rpm. Then, phase B was added to and dispersed in phase A. Phase C was prepared by dissolving a neutralizing agent and a sunscreen agent in purified water. Then, phase C was added to the mixture of phase A and phase B, mixed uniformly, and then defoamed.

### Experimental Example 1: Formulation and viscosity evaluation

The formulations and viscosities of the compositions according to the Examples and the Comparative Examples were measured, and the results are shown in Table 2 below. Viscosity measurement was performed using a Brookfield Viscosmeter LVT with spindle No. 4 and a spindle speed of 6 rpm.

**[Table 21**

| | Viscosity (cP) | Formulation |
|---|---|---|
| Example 1 | 72,000 | Gel form |
| Example 2 | 65,000 | Gel form |
| Example 3 | 50,000 | Gel form |
| Comparative Example 1 | 4,000 | Fluid and flowing form |
| Comparative Example 2 | 170,000 | Viscosity is too high to be dispersed |
| Comparative Example 3 | 8,000 | Paste, not gel |
| Comparative Example 4 | 16,000 | Fluid and flowing form |
| Comparative Example 5 | Not measurable | Impossible to form formulation |
| Comparative Example 6 | 30,000 | Paste, not gel |
| Comparative Example 7 | 15,000 | Fluid and flowing form |

As can be seen in Table 2 above, Examples 1 to 3 had a viscosity of about 50,000 to 80,000 cP, and were in a stable gel form. Representatively, the formulation form of the composition of Example 1 is shown in FIG. 1. On the other hand, Comparative Examples 1 to 7 did not stably maintain the viscosity and were not in gel form.

Specifically, in the case of Comparative Example 1 in which the content ratio of the sunscreen agent to the thickeners was extremely lower than that in the Examples, the composition was in a fluid liquid state due to its very low viscosity, and in the case of Comparative Example 2 in which the ratio of the sunscreen agent to the thickeners was higher than that in the Examples, dispersion was difficult due to a very high viscosity.

In addition, in the case of Comparative Example 3 in which the ratio of the first thickener to the second thickener was extremely lower than that in the Examples, the composition was in a paste state, not a gel state, due to its very low viscosity, and in the case of Comparative Example 4 in which the ratio of the first thickener to the second thickener was higher than that in the Examples, the composition was in a fluid and flowing form due to its low viscosity.

In addition, in the case of Comparative Example 5 in which the "PEG-150/decyl alcohol/SMDI copolymer" was used as the second thickener, the viscosity could not be measured because the formulation could not be formed, and a foreign body in the form of a white gum was formed immediately after the sunscreen agent was added. In addition, in the case of Comparative Example 6 to which the second thickener was not added, the composition was in a paste form, not a gel form, and in the case of Comparative Example 7 to which the first thickener was not added, the composition was in a fluid liquid state due to its low viscosity.

### Experimental Example 2: Evaluation of feeling of use

In order to evaluate the feeling of use of the compositions according to the Examples and the Comparative Examples, 30 women in their 20s and 40s were asked to evaluate items, including an initial moisturizing feeling, adhesion speed, stickiness after drying, and softness after drying, and the results are shown in Table 3 below.

In the experimental method, 1 mg/cm² of a sample was taken from the Examples and Comparative Examples and applied to a 3 cm (width) × 2 cm (length) area on the inside of the arm of each subject according to the usual makeup habit, and then each evaluation item was evaluated in 5 stages and scored in units of 1 point according to each stage on a 5-point scale. The feeling of use was evaluated by obtaining the average value of the sum of the scores of 10 people and then excluding the deviation.

### <Evaluation items>

- Initial moisturizing feeling: the higher the moisturizing feeling, the higher the score.
- Adhesion speed: the higher the adhesion speed, the higher the score.
- Stickiness after drying: the lower the stickiness and the higher the freshness, the higher the score.
- Softness after drying: the better the softness, the higher the score.

**[Table 3]**

| | Initial moisturizing feeling | Adhesion speed | Stickiness | Softness |
|---|---|---|---|---|
| Example 1 | 4.9 | 4.8 | 4.5 | 4.5 |
| Example 2 | 4.9 | 4.8 | 4.9 | 4.8 |
| Example 3 | 4.5 | 4.9 | 4.5 | 4.8 |
| Comparative Example 1 | 4.6 | 1.3 | 3.8 | 0.5 |
| Comparative Example 2 | 0.1 | 4.0 | 0.2 | 0.3 |
| Comparative Example 3 | 4.2 | 2.4 | 3.6 | 0.9 |
| Comparative Example 4 | 3.4 | 2.3 | 1.7 | 3.7 |
| Comparative Example 5 | - | - | - | - |
| Comparative Example 6 | 1.5 | 4.1 | 3.0 | 2.1 |
| Comparative Example 7 | 2.4 | 3.7 | 2.1 | 2.5 |
| 1: very bad, 2: bad, 3: moderate, 4: good, 5: very good | | | | |

As can be seen in Table 3 above, the compositions of Examples 1 to 3 obtained high scores in all the items. That is, it can be confirmed that the compositions of Examples 1 to 3 have an excellent moisturizing feeling and good skin adhesion, and may give a soft feeling without being sticky after drying.

On the other hand, in the case of Comparative Example 1 in which the ratio of the sunscreen agent to the thickeners was extremely lower than that in the Examples, the formulation did not adhere well to the skin and flowed on the skin. In the case of Comparative Example 2 in which the ratio of the sunscreen agent to the thickeners was higher than that in the Example, the formulation had no moisturizing feeling and was severely sticky after adhesion.

In addition, in the case of Comparative Example 3 in which the ratio of the first thickener to the second thickener was extremely lower than that in the Examples, the formulation gave a very hard feeling after drying, and in the case of Comparative Example 4 in which t the ratio of the first thickener to the second thickener was higher than that in the Examples, the formulation did not adhere well to the skin and was slippery.

In addition, in the case of Comparative Example 5 in which the "PEG-150/decyl alcohol/SMDI copolymer" was used as the second thickener, the formulation could not be completed and thus the feeling of use could not be tested.

In addition, in the case of Comparative Example 6 to which the second thickener was not added, the composition had a very hard feeling after drying, and in the case of Comparative Example 7 to which the first thickener was not added, the composition did not adhere well to the skin and flowed on the skin.

### Experimental Example 3: Evaluation of Long-term stability

Long-term stability evaluation of the compositions according to the Examples and the Comparative Examples was performed, and the results are shown in Table 4 below. Formulation stability was evaluated by placing each composition in a transparent plastic bottle container, maintaining each composition at temperatures of 25°C, 45°C and CYC (-5°C to 40°C), and observing temperature-dependent changes in the viscosity of each composition after one month.

**[Table 4]**

| | Change in viscosity |
|---|---|
| Example 1 | No change |
| Example 2 | No change |
| Example 3 | No change |
| Comparative Example 1 | Syneresis and phase separation occurred at all temperatures |
| Comparative Example 2 | Hardened like rubber at all temperatures |
| Comparative Example 3 | After 2 weeks or more, the viscosity increased by more than 40% compared to the existing ones under high temperature and CYC conditions |
| Comparative Example 4 | Viscosity increased while showing a lumpy appearance |
| Comparative Example 5 | Not measurable |
| Comparative Example 6 | Viscosity increased. |
| | Cracking of contents occurred at high temperature after 2 weeks or more |
| Comparative Example 7 | Viscosity increased while showing a lumpy appearance |

As a result, it was confirmed that the compositions of Examples 1 to 3 did not change in viscosity and were maintained in a very stable form for a long period of time.

On the other hand, in the case of Comparative Example 1, syneresis and phase separation occurred at all the temperatures, and the composition of Comparative Example 2 was hardened like rubber at all the temperatures. In the case of Comparative Example 3, the viscosity significantly increased over time, and in the case of Comparative Examples 4 and 7, a phenomenon occurred in which the viscosity increased while a crumpled appearance appeared. Comparative Example 5 could not be tested because the formulation could not be formed, and in the case of Comparative Example 6, a phenomenon occurred in which the contents were cracked at the high temperature. As such, it could be confirmed that, in the case of the compositions of the Comparative Examples, the composition was not stably maintained for a long time while the viscosity thereof greatly changed.

### Experimental Example 4: Measurement of sun protection factor (SPF)

The sun protection factor (SPF) values of the cosmetic compositions according to Examples 1 to 3 and Comparative Examples 1 to 4 and 6 to 7 were evaluated. Specifically, in order to evaluate the sun protection factor (SPF) values, 1.3 mg/cm² of a sample was taken from each composition and applied to a polymethyl methacrylate (PMMA; HELIO PLATE HD6) plate for about 30 seconds, and the applied sample was dried in the dark for 15 minutes. The SPF value of each dried sample was measured using the Labsphere UV 2000S *in vitro* SPF analyzer. The sun protection factor value was expressed as the average value of three experimental values, and the results are shown in Table 5 below.

**[Table 5]**

| | SPF value | | SPF value |
|---|---|---|---|
| Example 1 | 43 | Comparative Example 1 | 12 |
| Example 2 | 53 | Comparative Example 2 | 20 |
| Example 3 | 50 | Comparative Example 3 | 28 |
| | | Comparative Example 4 | 25 |
| | | Comparative Example 5 | - |
| | | Comparative Example 6 | 24 |
| | | Comparative Example 7 | 22 |

As a result, it could be confirmed that the compositions of Examples 1 to 3 had a high sun protection factor (SPF) value of 40 or higher. On the other hand, it could be confirmed that Comparative Examples 1 to 4, 6 and 7 had a significantly low sun protection factor (SPF) value because the function of the sunscreen agent could not be maintained, and in the case of Comparative Example 5, it was impossible to the SPF value because the formulation was not formed.

As can be seen from the above results, the sunscreen cosmetic composition in water dispersion form according to the present invention has a high moisturizing feeling, a fresh and excellent feeling of use, and high UV protection ability, and may provide a stable gel formulation.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A sunscreen cosmetic composition comprising
a water-soluble sunscreen agent, and
different kinds of thickeners which consist of a first thickener and a second thickener,
wherein the first thickener is an inorganic thickener, the second thickener is an organic thickener, and the cosmetic composition is in aqueous dispersion form.

2. The sunscreen cosmetic composition according to claim 1, wherein the first thickener is a silicate mineral.

3. The sunscreen cosmetic composition according to claim 1, wherein the second thickener is at least one selected from among acrylic thickeners, polysaccharide thickeners, and derivatives thereof.

4. The sunscreen cosmetic composition according to claim 1, wherein the first thickener comprises at least one selected from the group consisting of hectorite, bentonite, magnesium aluminum silicate, saponite, montmorillonite, smectite, kaolin, illite, saponite, and mixtures thereof.

5. The sunscreen cosmetic composition according to claim 1, wherein the second thickener comprises at least one selected from the group consisting of carbomer, an acrylate/C10-30 alkyl acrylate crosspolymer, an acrylic acid/Vp crosspolymer, hydroxyethyl cellulose, hydroxypropyl cellulose, hydroxypropyl methyl cellulose, hydroxypropyl methyl cellulose stearoxy ether, cellulose gum, xanthan gum, tara gum, sclerotium gum, Acacia Senegal Gum, gellan gum, guar gum, ethyl cellulose, cellulose, carboxymethyl hydroxyethyl cellulose, carboxymethyl cellulose, methyl hydroxyethyl cellulose, hydrolysates thereof, and mixtures thereof.

6. The sunscreen cosmetic composition according to claim 1, wherein the water-soluble sunscreen agent comprises at least one selected from the group consisting of terephthalylidene dicamphor sulfonic acid, phenylbenzimidazole sulfonic acid, disodium phenyl dibenzimidazole tetrasulfonate, benzophenone-4, benzophenone-5, and mixtures thereof.

7. The sunscreen cosmetic composition according to claim 1, wherein a content ratio of the water-soluble sunscreen agent to the different kinds of thickeners is 1: 0.02 to 0.36.

8. The sunscreen cosmetic composition according to claim 1, wherein a content ratio of the first thickener to the second thickener is 1: 0.1 to 30.0.

9. The sunscreen cosmetic composition according to claim 1, wherein the first thickener is contained in an amount of 0.005 to 2.5 wt% based on the total weight of the composition.

10. The sunscreen cosmetic composition according to claim 1, wherein the second thickener is contained in an amount of 0.3 to 3.0 wt% based on the total weight of the composition.

11. The sunscreen cosmetic composition according to claim 1, wherein the water-soluble sunscreen agent is contained in an amount of 0.5 to 28.0 wt% based on the total weight of the composition.

12. The sunscreen cosmetic composition according to claim 1, wherein the composition is in a gel form.

13. The sunscreen cosmetic composition according to claim 1, wherein the composition has a viscosity of 35,000 to 150,000 cP.
